# EUROPEAN PATENT APPLICATION

(11) **EP 4 813 437 A1**
(43) Date of publication of application: **30.09.2026**
(21) Application number: 25167103.8
(22) Date of filing: 28.03.2025
(51) Int. Cl.: A61M 37/00

(54) **METHOD AND SYSTEM FOR MANUFACTURING A MICRONEEDLE ARRAY, AS WELL AS CARRIER AND MICRONEEDLE ARRAY**

(71) Applicant: LTS Lohmann Therapie-Systeme AG, 56626 Andernach (DE)
(72) Inventor: SCHERR, Sebastian, 56335 Neuhäusel (DE); KUCH, Christopher, 56410 Montabaur (DE); HEIDARY DASTJERDI, Maral, 53424 Remagen (DE); SCHLÜTER, Anna, 53639 Königswinter (DE); BÖHM, Rolf, 56271 Kleinmaischeid (DE); DONNELLY, Ryan Francis, Newry, BT34 2FL (IE); ANJANI, Qonita Kurnia, Belfast (GB)
(74) Representative: dompatent

(57) **Abstract**

The invention relates to a method for manufacturing a microneedle array (10) comprising a carrier (14) and a plurality of microneedles (12) connected to the carrier (14), the method comprising the steps of: molding the plurality of microneedles (12) in a mold (16), providing the carrier, and attaching the carrier (14) to the plurality of microneedles (12) in the mold (16). The invention further relates to a system for manufacturing a microneedle array (10), a carrier (14) and a microneedle array (10).

## Description

### TECHNICAL FIELD OF THE INVENTION

The present invention relates to a method and a system for manufacturing a microneedle array, as well as a carrier and a microneedle array.

The present invention is generally in the field of microneedles for the transport of therapeutic, diagnostic, cosmetic, biological or other molecules into, out of or across the skin or other tissue barriers.

### BACKGROUND OF THE INVENTION

Microneedles might be used to deliver a wide variety of substances of interest directly into the skin. For this purpose, the microneedles are just long enough to penetrate only the outer skin layers (e.g. *stratum corneum* ~10-20 µm and epidermis ~50-150 µm), but preferably do not reach nerves (nociceptors) and blood vessels in dermis layer, therefore being relatively pain-free in doing so. However, their small size may hinder other factors that are important for their functionality and/or manufacture. This is particularly true in the case of producing a microneedle patch for transdermal drug delivery.

For example, since microneedles are short in length in comparison to the base or backing to which they are affixed to, tissue insertion can be difficult. This results from the elastic nature of the targeted tissue (e.g., skin) because much of the applied force when administering them to skin is used to deform the skin underneath the entirety of the microneedle patch in order for the microneedles to sufficiently contact and penetrate the tissue. Therefore, the patch application force required for successful microneedle insertion can be higher than the force to insert the microneedles alone. This has resulted in the development of complex applicators that apply microneedle patches to the skin with impact. This adds cost and complexity, which are undesirable.

Conventional molding methods resulting in monolayer or multi-layer microneedie arrays generally are not well suited for making microneedle arrays in a simple, fast, highly reproducible, accurate and cost-effective manner.

After molding, the microarray consisting of the microneedles connected to the carrier must be cured, particularly dried. The drying process requires a lot of time and energy. For example, in an exemplary process of the state of the art, curing the microarray, particularly drying out the solvent, such as ethyl acetate, from the molding material, particularly from the molding material for the carrier, takes about 24 hours, whereby the microarray is dried at different temperatures, for example up to 80° C, for different amounts of time. Said process thus might result in deformation of microarray patches manufactured thereof. Lastly, said process influences the costs as well as the quality and performance of microneedle arrays produced thereof.

Further problems of carriers of the state of the art are, for example, a diffusion of substances, particularly active ingredients, into the carrier. This may lead to false active ingredient delivery properties, corruption of the carrier and/or lack of content uniformity of final product.

In sum, there remain needs to improve microneedle designs for better tissue insertion and to improve microneedle manufacturing methods, particularly for such improved designs.

Thus, an object of the present invention is an optimized method and system for manufacturing a microneedle array, as well as an optimized microneedle array.

Further object is to provide a pre-manufactured carrier for providing a better mechanical support for the plurality of microneedles connected thereto and improved insertion ability of the microneedle array.

### DETAILED DESCRIPTION

Generally, microneedles have a length of 25 to 2000 µm, preferably between about 200 µm and 1200 µm, and most preferably between about 500 µm and 1000 µm.

The volume of a microneedle can be between about 100 pl and 100 nl. In most cases, it is between about 5 nl and 20 nl.

A plurality of the microneedles is usually assembled as a microarray, whereby the microneedles are arranged on a carrier, such as a patch, plaster, backing or base plate, in any suitable density. For example, a plurality of microneedles may be arranged in uniformly or staggered rows in an array, wherein each microneedle is separated from its nearest neighboring microneedle by a distance about equal to the height of the microneedle.

The carrier can have different sizes. Typically, carriers are used in the size of a thumb, preferably with a surface area of 1-2 cm². Microarrays often have a high number of microneedles, for example, 100 to 600 microneedles per cm². The number of needles on the carrier is limited by the natural resistance of the skin. The greater the number of needles on a carrier, the more force is required to apply the microneedle array into the skin.

A microneedle array may include different microneedles, for example containing different compositions of materials, including different active ingredients and/or excipients and/or other materials.

The microneedles may be dissolvable microneedles, hydrogel microneedles, hollow microneedles or coated microneedles. Dissolvable microneedles are typically formed from water-soluble materials and dissolve after being administered to the skin. Hydrogel microneedles are typically formed from a hydrogel polymer. Usually, hydrogel microneedles swell after being administered. As they absorb bodily fluids, they can be used for both delivery of bioactive substances, and capture of bodily fluids for diagnosis, for example. It is conceivable that the hydrogel microneedles are connected to a reservoir with the active ingredient. Hollow microneedles are microneedles with a hollow core; they can be used to deliver bioactive substances or extract fluids.

Another concept for delivering active ingredients using microneedles is to coat, for example, solid and/or non-dissolvable microneedles with the active ingredient. Upon insertion of the microneedle into the skin, the active ingredient can be released, for example, dissolved, from the microneedle and thus be delivered into the skin.

Furthermore, microneedles of the state of the art can be made of fully or partially dissolvable materials containing the active ingredient. For example, biocompatible polymers are used in dissolving microneedles, which form the matrix for the active ingredient. Once the microneedles have penetrated the skin, they dissolve and release the active ingredient into the skin. The polymer, which is also absorbed, breaks down in the body and is excreted. After insertion, the microneedles can be applied while being connected to the carrier or, after insertion, the needles can be detached, for example, broken off or dissolved, from the carrier.

Dissolvable microneedles can have multiple layers, particularly comprise different materials per layer. For example, a tip of the microneedle can comprise the active ingredient, while the rest of the microneedle is free of active ingredient. Furthermore, it is possible to implement layers having different dissolving properties and/or comprising different active ingredients.

Often, the microneedles, particularly dissolvable microneedles, are integrally molded with the carrier. For example, one or more molding materials are dispensed into the cavities of the mold, whereby the cavities correspond to negatives forms of the microneedles to be produced. Subsequently, a further, often non-active ingredient containing material is deposited onto the microneedle material to mold the carrier directly connected to the microneedles.

### SUMMARY OF THE INVENTION

The objects of the invention are solved by a method according to claim 1, a system according to claim 11, a carrier according to claim 14 and a microneedle array according to claim 19.

The invention relates to a method for manufacturing a microneedle array comprising a carrier and a plurality of microneedles connected to the carrier. The method comprises the step of molding the plurality of microneedles in a, preferably gas-permeable, mold. A further step of the method is to provide the carrier. The carrier provides mechanical support for plurality of microneedles connected thereto and is preferably solid. The term "solid" as used herein means the carrier is generally not hollow or filled with empty spaces. In another embodiments carrier might be flexible as well as solid. The carrier is preferably one or more of the following, solid, non-viscous, cured and/or hardened.

It is preferred that the carrier is pre-manufactured.

Preferably, the carrier is insoluble in water. In some embodiments the carrier is hydrophobic. Advantageously, a hydrophobic carrier prevents migration of hydrophilic substances from the microneedles into the carrier.

Preferably, the method comprises the step of manufacturing the carrier. The step of manufacturing the microneedles may be performed before, after or during the step of manufacturing the carrier. A further step is to connect, particularly attach, the carrier to the plurality of microneedles in the mold. Preferably, the carrier is joined, particularly through material bonding, to the microneedles. The carrier is preferably attached to a base of the microneedles. The method preferably comprises the step of connecting the carrier to the microneedles to attach the carrier to the microneedles. It is preferred that the carrier is attached, particularly connected, to the microneedles by a transport device such as a vacuum gripper. Molding the microneedles preferably comprises the step of filling a molding material into the mold, particularly to fully fill the mold, whereby filling is preferably performed by dispensing the molding material into the mold. The mold preferably has a plurality of cavities for molding the microneedles. Preferably, the mold is a matrix. Particularly, the cavities have a cone shape; a pyramid shape; an obelisk shape; or a cylinder shape having, for example a circular, triangular or rectangular base. The molding material is preferably filled into the cavities, particularly into all cavities. It is preferred that the cavities are fully filled or overfilled with the molding material, whereby this filling state relates to the liquid or solid state of the molding material. The molding material can comprise a plurality of different mediums, preferably materials, more preferably compounds. If, in a preferred embodiment, multi-layer microneedles should be produced, the molding material can comprise multiple materials which are subsequently filled into the cavities. Preferably at least one of the materials to be filled into the cavities comprises an active ingredient, particularly the material to form a tip and/or core of the microneedies. At least one material to be filled into the cavities may be free of active ingredient, particularly the material of the shell and/or a ground layer and/or a middle layer of the microneedles. The microneedles and/or carrier may particularly have one or more features of the microneedles and/or carrier described in the introduction in connection with the state of the art. The carrier preferably consists of a, particularly completely, dried material and/or the carrier is solid. The structure of the carrier can be flexible. The carrier preferably corresponds to a backing, for example a backing layer or backing plate, of the microneedles. Particularly, the carrier corresponds to a chip of the microneedle array.

It is preferred that the carrier is adhesively attached to the, preferably fully or partially cured, particularly dried, microneedles. For example, an adhesive can be provided to the microneedles, particularly on a base of the microneedles, and/or to the carrier. Preferably the microneedles, particularly the base of the microneedles, and/or the carrier comprises an adhesive, for example an adhesive layer. For example, the method can comprise the further step of applying an adhesive, particularly an adhesive layer, to the microneedles and/or the carrier.

It is preferred that the step of attaching the carrier to the microneedles comprises, particularly consists of, the step of bringing the carrier into contact with the microneedles in the mold. The step of bringing the carrier into contact with the microneedles preferably comprises pressing the carrier onto the microneedles. Preferably, the carrier is moved, for example by a transport device such as a vacuum gripper, to come into contact with the microneedles. It is preferred that a force is applied to the carrier, particularly by the transport device, when bringing the carrier into contact with the microneedles and preferably the force is transmitted to the mold and/or the microneedles.

It is preferred that the method comprises the further step of demolding the microneedles from the mold by removing the carrier with the attached microneedles from the mold. It is preferred that the carrier is pressed onto the microneedles and removed by a transport device such as a vacuum gripper. Particularly, the step of demolding is performed within a maximum of 10 seconds, preferably within a maximum of 5 seconds, more preferably within a maximum of 1 second after attaching the carrier to the microneedles. It is preferred that the carrier is moved to be brought into contact with the microneedles and subsequently, for example within a maximum of 10 seconds, preferably within a maximum of 5 seconds, more preferably within a maximum of 1 second, is removed with the microneedles attached from the mold thereby demolding the microneedles. Preferably, the method comprises the further steps of packaging the microneedle array after demolding.

It is preferred that the carrier is attached to the microneedles by connecting the carrier to the microneedles while the microneedles are in a liquid state. Particularly, the microneedles are not cured, at least not fully cured yet. It is thus preferred that the carrier is attached to the microneedles directly after molding, particularly within a maximum of 15 minutes, or within a maximum of 5 minutes, or within a maximum of 1 minute, or within a maximum of 10 seconds or within a maximum of 1 second after molding, preferably of filling, more preferably fully filling the mold.

It is preferred that the carrier is attached to the microneedles by diffusion bonding and/or welded joining. This diffusion bonded and/or welded joined connection between the carrier and the microneedles is preferably achieved by connecting the carrier to the microneedles in a liquid state, preferably in a state in which the microneedles are not cured, at least not fully cured. The carrier is particularly pressed onto the microneedles. Preferably, the connection of the carrier and the microneedles is achieved by a bond formed through molecular interaction and/or diffusion. Particularly, the attaching step may comprise that the carrier is connected to the microneedles, whereby the temperature of the microneedles, particularly of the molding material, and the material of the carrier are configured such that the carrier, particularly partly, melts, thereby creating a welded seam between the microneedles and the carrier.

It is preferred that the carrier is a backing plate having a rectangular, square, round or oval shape. Particularly, the carrier can have a diameter, particularly a maximum diameter, of 10 mm to 50 mm, more preferably of 10 mm to 30 mm, for example of 10 mm.

The microneedles may comprise an active ingredient such as a drug or other cosmetic or health-related substance and combinations thereof. The drug may be a small molecule drug, peptide, antibody, nucleic acid, cells or vaccines or combinations thereof. Advantageously, application of the microarray to the skin thereby administers the active ingredient to the recipient through the skin. Particularly, the microneedles comprise a polymer. The microneedle may comprise 45-99%, 55-95%, 65-85%, or 75-80% w/w polymer, with the remainder being water and other substances. The polymer may be polyvinyl alcohol, polyvinylpyrrolidone, polycaprolactone, polylactic acid, poly(ester amide), poly(lactic-co-glycolic acid), collagen, hydroxyapatite, hyaluronic acid, dextran, chitosan or combinations thereof. Preferably, the microneedle dissolves within 15-120 min, or 30-60 min after application on the skin.

Preferably, the carrier comprises or consists of biocompatible material, for example, ceramic, metal and/or plastic, particularly inert plastic. The term " biocompatible" refers natural or synthetic materials that can function in close contact with living tissue or even substitute a part of a living system. Furthermore, biocompatible material is not causing any harmful effects when it is in contact with the body, i.e. material is nonirritant, nontoxic, nonthrombogenic, noncarcinogenic, etc. Biocompatible materials are designed to interface with biologically active systems for evaluating, treating, augmenting, or substituting any tissue, organ, or function of the body.

The biocompatible materials are compatible with U.S. Food and Drug Administration (FDA) requirements and fulfill the ISO 10993 (the international standard for device testing) provides guidance on how biocompatible materials can be used and tested as part of wider risk management and risk-reduction strategy. Preferred material for the carrier are:
1. Fast dissolving materials, such as:
   - Dextran, which preferably has the characteristics of: polysaccharide, biocompatible and/or dissolves quickly in aqueous media.
   - PVP (polyvinylpyrrolidone), which preferably has the characteristics of: water-soluble, biocompatible, widely used in the pharmaceutical industry, and/or dissolves on contact with moisture.
   - Gelatin, which preferably has the characteristics of: natural protein, water-soluble, and/or biodegradable.
   - Alginate, which preferably has the characteristics of: polysaccharide from algae, water-soluble, and/or biocompatible.
   - Sugar, for example sucrose or mannitol, which preferably has the characteristics of: rapid dissolution in aqueous media.
2. Slow-dissolving materials, such as
   - PLGA (polylactide-co-glycolide), which preferably has the characteristics of: biocompatible, and/or biodegradable.
   - PEA (polyester amide) which preferably has the characteristics of: biocompatible, and/or biodegradable.
   - PCL (polycaprolactone).
   - Chitosan, which preferably has the characteristics of: biodegradable, slow dissolution in an acidic environment, and/or can be chemically modified to control dissolution.
   - Starch-based polymer, which preferably has the characteristics of: biocompatible, and/or slow dissolution depending on the cross-linking.

Further possible preferred materials for the carrier can be: MED610, PET, PLA (polylactic acid), PHA (polyhydroxyalkanoates), cellulose acetate, chitosan and/or alginate.

The carrier preferably has a smooth or structured surface, particularly a smooth or structured connection surface for connection to the microneedles.

Particularly, the carrier has a thickness of 50 µm to 1000 µm, preferably of 100 µm to 700 µm and more preferably of 200 µm to 500 µm.

It is preferred that, the method comprises the further step of manufacturing the carrier by cutting, preferably die cutting, the carrier from a base layer, particularly a film or a mat; or by molding such as injection molding; or by 3D printing. For example, the carrier can be cut from a film, for example a PET film, particularly a PET film roll. The film preferably has one or more properties of the carrier. Particularly, the film has a thickness of 50 µm to 1000 µm, preferably of 100 µm to 700 µm and more preferably of 200 µm to 500 µm. Preferably, a curing step is performed after manufacturing, particularly 3D printing, the carrier. Curing can be induced at least by heat, radiation, electron beams, or chemical additives. The carrier may particularly be molded individually using a single dosing process. In this case, a drying step, for example in a temperature oven and/or drying under vacuum may be performed.

If the carrier is manufactured by injection molding, preferred materials for the carrier are PLA, PHA or other biocompatible polymers. Injection molding is a good method for mass production. Preferably, the biocompatible material is heated, injected into a mold and cooled. Particularly, precise tooling can be applied to produce complex geometries, including cylindrical carriers. However, other shapes for the carrier are also possible. Preferably, in view of sustainability, the use of biobased or recycled plastics is possible.

If the carrier is manufactured by 3D printing, also referred to as additive manufacturing, preferred materials are biocompatible and biodegradable materials such as PLA, bioactive polymers or modified bioinks. Preferably, materials for the carrier are biocompatible polymers such as MED625FLX, MED610 and MED620. The materials are suitable for over 30 days of skin contact and up to 24 hours of mucosal contact. MED625FLX (Stratasys) is flexible, transparent material and allows the direct printing of objects. MED610 (Stratasys) is solid and transparent material. VeroGlaze MED620 (Stratasys) is an opaque, rigid material for precise models and customized applications. Further product features include IZOD impact strength of 20 J/m - 30 J/m, heat deformity temperature 45 °C - 50 °C, flexural strength 75 Mpa - 110 MPa, tensile strength 50 Mpa - 65 MPa.

Fused Deposition Modeling (FDM) or stereolithography (SLA) may be used. Advantages of 3D printing are, for example, minimal material waste and rapid iterations.

The carrier may also be manufactured by microfabrication techniques. Preferred materials for the carrier in microfabrication techniques are silicon, hydrogelbased materials or other biocompatible polymers. Lithography, microinjection molding or hot embossing may be performed.

The carrier may also be manufactured by extrusion methods. Preferred materials for the carrier in extrusion methods are biocompatible polymers such as PCL (polycaprolactone) or PLA. For example, extrusion can be used to produce cylindrical structures with controlled dimensions. These structures can then be cut or further processed to manufacture the carrier.

The carrier may also be manufactured by casting and thermoforming. Preferred materials for the carrier in casting and thermoforming are biopolymers such as gelatin, alginate or modified starches. Preferably, liquid material is cast into a mold and allowed to harden. Particularly, compostable and/or water-soluble materials are possible.

The carrier may also be manufactured by a MeltPrep process. The MeltPrep process, particularly the Vacuum Chamber Molding process, is based on heating a thermoplastic, such as biocompatible material, to its melting point and then forming the desired structures in a mold. Biocompatible and biodegradable polymers such as PLGA, PCL, or starch-based polymers can be used. Preferably, no solvents are used, which makes the process sustainable.

A preferred process to manufacture the carrier by the MeltPrep process is:
1. Material preparation:
   Biocompatible materials such as PCL, PLGA, or other polymers can be used.
2. Melting process:
   The material is heated until it is soft enough to fill the mold.
3. Filling the mold:
   Pressure is applied to force the molten mass into the mold.
4. Cooling and demolding:
   After cooling, the finished workpiece (e.g. a cylindrical chip or microneedles) is removed from the mold.

Possible materials:
- PLGA and/or PCL: Slow-dissolving and robust;
- PVP and/or dextran: for quick-dissolving applications; and/or
- Biobased polymers such as polysaccharides or modified starches.

The carrier may also be manufactured by CNC machining. Preferred materials for the carrier in CNC machining are biocompatible plastics (e.g. PEEK) or sustainable materials such as cellulose composites.

The carrier may also be manufactured by powder bed method, such as SLS or MJF. Preferred materials for the carrier in powder bed methods are biocompatible powders such as polyamide based on castor oil.

Preferably, for example, depending on the production volume, complexity and specific requirements (e.g. biocompatible coatings), these methods can be combined.

It is preferred that, a vacuum is applied to the mold, particularly an outer surface of the mold, at least during the step of molding the plurality of microneedles. The vacuum is preferably applied such that the molding material is drawn into the cavities of the mold.

Preferably, the carrier is attached to microneedles, which are located in several separate molds. Thus, it is preferred that the carrier is attached to multiple sets of a plurality of microneedles. As such, the carrier corresponds to a carrier for multiple sets of a plurality of microneedles. Particularly, the carrier having the multiple sets of a plurality of microneedles can be separated, for example, by cutting, to segregate individual microneedle arrays therefrom.

Particularly, the carrier is connected, preferably integrally or adhesively, to an applicator punch for a microneedle applicator, or to a, particularly flexible, microneedle array patch. The method preferably comprises, particularly after manufacturing the carrier, the step of attaching the carrier to the punch or to the patch. Preferably, the step of attaching the carrier to the microneedles is performed by connecting the carrier connected to the punch to the microneedles, or by connecting the carrier connected to the patch to the microneedles. As such, it is preferred that a unit comprising the carrier attached to the punch, or carrier attached to the patch is moved and the carrier as part of the unit is attached to the microneedles.

Preferably, the mold has a flat surface from which the cavities extend. Particularly a complete upper side of the mold is flat.

It is preferred that the method comprises the further step of curing, particularly at least partly curing, the microneedles within the mold. The step of curing is preferably performed before, after and/or during the step of attaching the carrier to the microneedles. It is preferred that the microneedles are cured for 1 minute to 60 minutes, or for 10 minutes to 45 minutes, or for 20 minutes to 40 minutes, for example for 30 minutes, particularly before and/or after the step of attaching the carrier to the microneedles. In the step of curing the microneedles and/or in the step of curing the carrier, curing preferably comprises drying and/or solidifying of the microneedles. Particularly curing comprise evaporating a thinner, for example a solvent, from the molding material of the microneedles and/or the carrier. It is preferred that a vacuum is applied to the microneedles and/or the carrier during curing, particularly during a majority or the complete during process. Curing can preferably comprise heating the microneedles and/or providing a gas flow to the microneedles and/or UV treatment of the microneedles. After curing, the microneedles and/or the carrier is preferably hardened and particularly solid.

The invention further relates to a system for manufacturing a microneedle array. The system is preferably adapted to perform one or more steps of the method according to the invention. The system comprises a device for attaching a carrier to the microneedles in the mold. Preferably the device is adapted to press the carrier onto the microneedles in the mold. The device for attaching the carrier is preferably a transport device, particularly a gripper, more preferably a gripper arm, such as a vacuum gripper.

It is preferred that the device for attaching the carrier is adapted to demold the microneedles by removing the carrier from the mold with the microneedles attached to the carrier.

The device for attaching the carrier is preferably adapted to receive, for example pick up, the carrier. Further, the device for attaching the carrier is adapted to move the carrier, particularly move the carrier to bring the carrier into contact with the microneedles in the mold and to move the carrier to remove the carrier from the mold. Further, the carrier can preferably be discharged, particularly for packaging, for example, the carrier can be placed into a packaging or in a packaging area by the device. Additionally, the device for attaching the carrier is preferably adapted to retain, for example fix, the carrier, and release the carrier.

It is preferred that the system further comprises a vacuum device for applying a vacuum to the mold. The vacuum device is preferably adapted to apply a vacuum to the outer surface of the mold and/or apply a vacuum to the mold such that the molding material is drawn into the cavities of the mold.

Preferably, the system further comprises a dispenser, particularly a single-drop dispenser, for dispensing a molding material for the microneedles to be produced into the mold.

According to the further aspect of the invention it is provided a carrier for providing a mechanical support for a plurality of microneedles connected, particularly attached, thereto, wherein the carrier is pre-manufactured and obtainable by process of cutting, injection molding or 3D printing. The term "pre-manufactured" may refer to process that involves the fabrication of carrier in a controlled environment before it is transferred and applied to a plurality of microneedles. Pre-manufacturing may be performed using different processes, such as cutting, injection molding or 3D printing. Advantageously, pre-manufacturing enables customization of carrier's size, shape and/or material used. Further advantageously, by pre-manufacturing carrier, the drying time for microneedle array is significantly shortened and thus subsequently the costs of manufacturing have been reduced. In addition, such pre-manufactured carrier may also be sterile. The carrier and/or the microneedles preferably comprises one or more features of the carrier and/or the microneedles as described in the method according to the invention.

The invention further relates to a microneedle array. The microneedle array is preferably manufactured by a method for manufacturing a microneedle array according to the invention and/or by a system for manufacturing a microneedle array according to the invention. The microneedle array comprises a carrier, preferably a backing plate. Further, the microneedle array comprises a plurality of microneedles. It is preferred that the microneedles are connected, particularly attached, to the carrier by adhesive, diffusion or welded joints. The carrier preferably corresponds to the carrier according to the invention.

According to the invention, preferably, the microneedles are dissolving microneedles or hydrogel microneedles. Particularly, the microneedles comprise one, two or three layers. The microneedles may comprise a shell and a core.

It is preferred that one or more objects according to the invention, i.e. the method, the system, the carrier and/or the microneedle array, comprise one or more features of the other objects according to the invention.

In the following the present invention is described in more detail with reference to the accompanying drawings.

The figures show:
- Figure 1: a sectional diagram showing a preferred embodiment of a method for manufacturing a microneedle array according to the invention and a preferred embodiment of a microneedle array according to the invention,
- Figures 2a-2d: schematic sectional views of preferred embodiments of a microneedle array according to the invention,
- Figures 3a-3c: flow charts indicating preferred embodiments of a method for manufacturing a microneedle array according to the invention,
- Figure 4: a photo of an exemplary, preferred production setup for manufacturing microneedle arrays,
- Figure 5: exemplary photos of preferred, manufactured microneedle arrays,
- Figure 6: an exemplary photo of a preferred carrier, mold and mold combined with carrier,
- Figure 7: an exemplary photo of a preferred packaging for a microneedle array, and
- Figures 8-9: photos of test results for exemplary, preferred microneedle arrays.

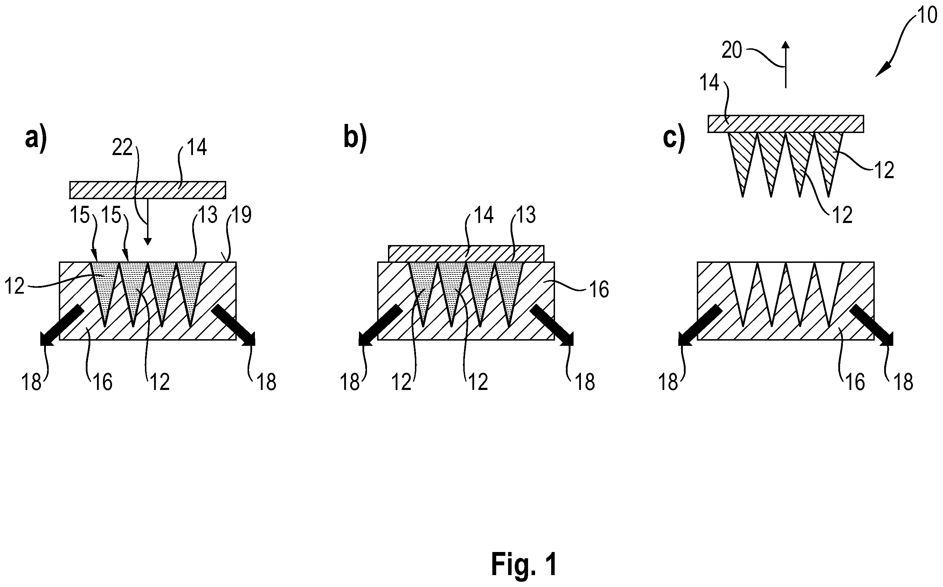

Figure 1 shows three states a)-c) of an embodiment of a method for manufacturing a microneedle array 10.

A gas-permeable mold 16 is provided having a plurality of cavities 15 for molding microneedles 12. The cavities 15 extend from a flat surface 19 of the mold 16 into the mold 16. The cavities 15 have conical shapes corresponding to negative forms of the microneedles 12 to be manufactured.

A vacuum (indicated by arrow 18) is applied to the gas-permeable mold 16 to draw molding material into the cavities 15.

As shown in section a), a molding material for producing the microneedles 12 is dispensed into the cavities 15.

A carrier 14 is moved (indicated by arrow 22) into contact with the flat surface 19.

As shown in section b), the carrier 14 is brought into contact with the mold 16 and thus in contact with the microneedles 12, particularly the base 13 of the microneedles 12. Particularly, the carrier 14 is pressed onto the mold 16 and thus onto the microneedles 12.

During this process, the carrier 14 is attached to the microneedles 12.

For example, the carrier 14 can be attached to the microneedles 12 by diffusion bonding and/or welded joining. This attaching can, for example, be achieved if the molding material of the microneedles 12 is still liquid and thus, a diffusion bonded and/or welded joined connection occurs between the microneedles 12 and the carrier 14.

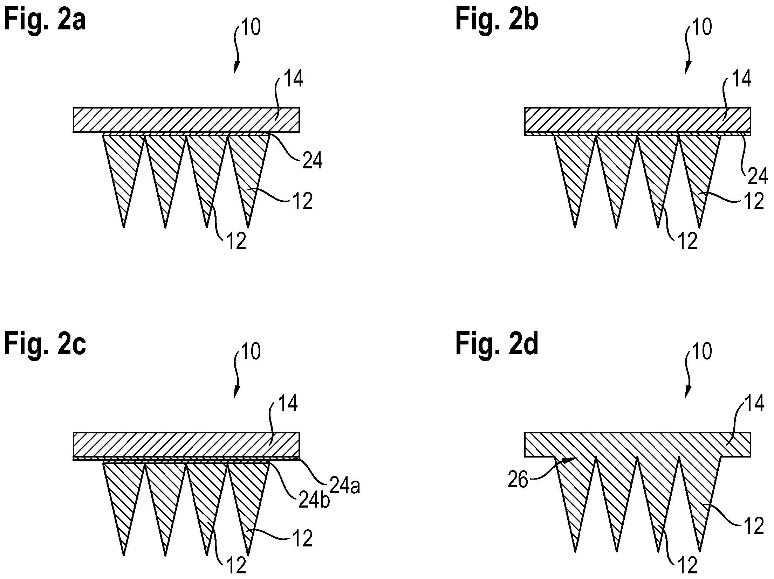

Alternatively, the carrier 14 can be attached to the, preferably cured, microneedles 12 by an adhesive (not shown in Fig. 1) 24 on the microneedles 12 (see Fig. 2a) and/or on the carrier 14 (see Fig. 2b). Thus, if the carrier 14 is brought into contact with the microneedles 12 an adhesive bond occurs, which attaches the carrier 14 to the microneedles 12.

As shown in section c), the carrier 14 is removed (indicated by arrow 20) from the mold 16, thereby demolding the microneedles 12 attached to the carrier 14 from the mold 16.

In this state, the microneedles 12 are preferably cured, particularly fully cured.

The microneedles 12 attached to the carrier 14 correspond to an embodiment of a microneedle array 10.

Figures 2a-2d show different preferred embodiments of a microneedle array 10.

Figure 2a shows a microneedle array 10, whereby an adhesive 24, for example an adhesive layer, was applied to the microneedles 12. When bringing the carrier 14 into contact with the microneedles 12, the carrier 14 was adhesively connected to the microneedles 12 by the adhesive 24 on the microneedles 12. Thus, Fig. 2a shows a microneedle array 10 whereby the microneedles 12 are attached to the carrier 14 by an adhesive 24 on the microneedles 12.

Figure 2b shows a microneedle array 10, whereby an adhesive 24, for example an adhesive layer, was applied to the carrier 14. When bringing the carrier 14 into contact with the microneedles 12, the carrier 14 was adhesively connected to the microneedles 12 by the adhesive 24 on the carrier 14. Thus, Fig. 2b shows a microneedle array 10 whereby the microneedles 12 are attached to the carrier 14 by an adhesive 24 on the carrier 14.

Figure 2c shows a microneedle array 10, whereby an adhesive 24a, for example an adhesive layer, was applied to the carrier 14 and an adhesive 24b, for example an adhesive layer, was applied to the microneedles 12. When bringing the carrier 14 into contact with the microneedles 12, the carrier 14 was adhesively connected to the microneedles 12 by the adhesives 24a, 24b. Thus, Fig. 2a shows a microneedle array 10 whereby the microneedles 12 are attached to the carrier 14 by an adhesive 24a, 24b on the microneedles 12 and on the carrier.

Figure 2d shows a microneedle array 10, whereby the connection between the carrier 14 and the microneedles 12 is achieved by a diffusion bonded and/or welded joined connection 26. As such, the carrier 14 is preferably integrally connected, particularly integrally merged, with the microneedles 12.

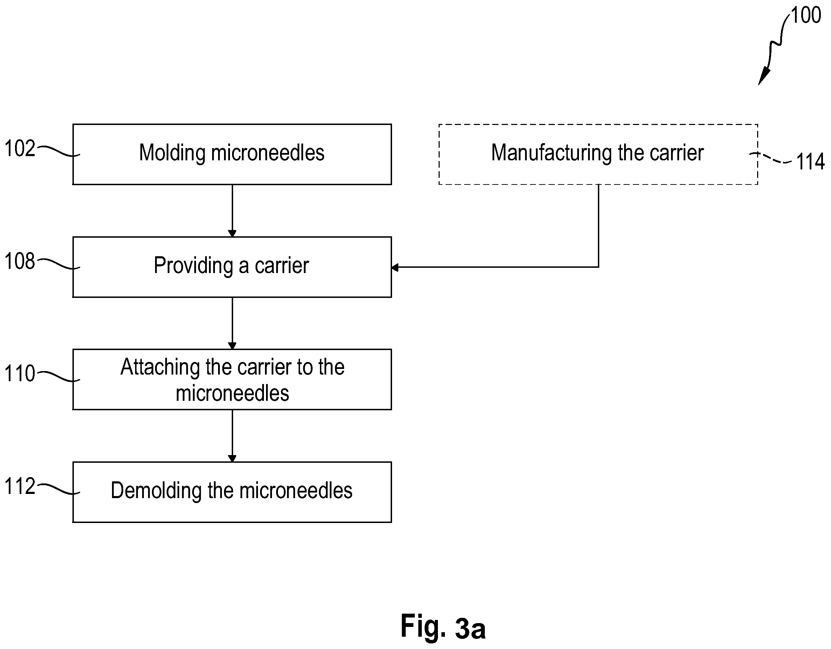

Figure 3a shows a flow chart indicating an embodiment of a method 100 for manufacturing a microneedle array 10.

A first step of the method 100 is molding the microneedles 12, preferably in a gas-permeable mold 16 *- step: Molding the microneedles 102.*

During, before or after the step of molding the microneedles 12, the carrier 14 is manufactured and then provided - *steps: Manufacturing the carrier 114, Providing the carrier 114.* The carrier 14 may, for example, be manufactured by cutting, such as die cutting, the carrier 14 from a film, particularly a PET film. Alternatively, the carrier 14 may, for example, be molded using injection molding, or may be 3D printed.

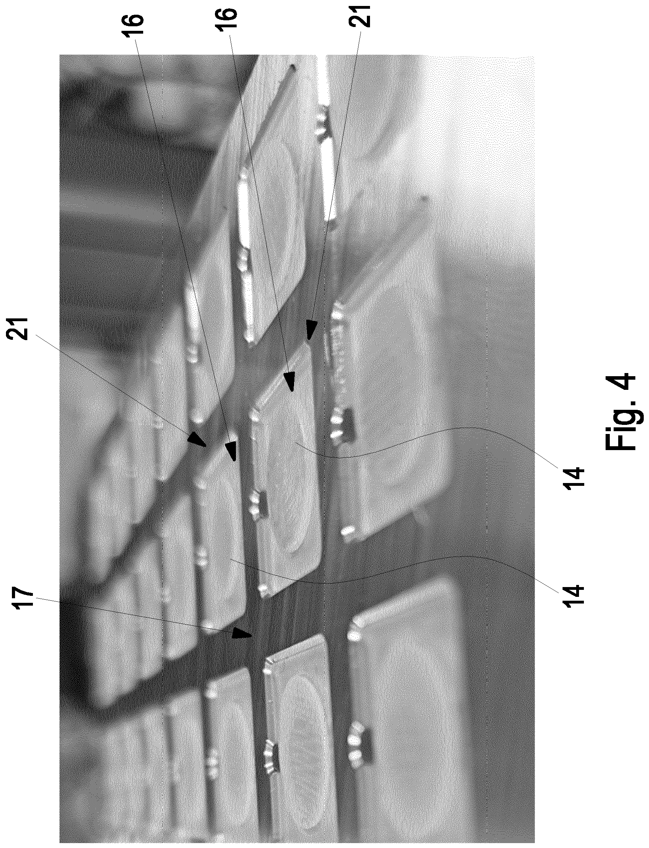

Next, the carrier 14 is brought into contact with the microneedles 12 and is thereby attached to the microneedles 12, particularly adhesively; or by a diffusion bonded and/or welded joined connection - *step: Attaching the carrier to the microneedles 110.* Figure 4a shows

Next, the microneedles 12 are demolded by the carrier 14, particularly by removing the carrier 14 with the microneedles 12 attached from the mold 16 - *step: Demolding the microneedles 112.*

Afterwards, the microneedles 12 attached to the carrier 14, which corresponds to an embodiment of a microneedle array 10, may be packaged (not shown).

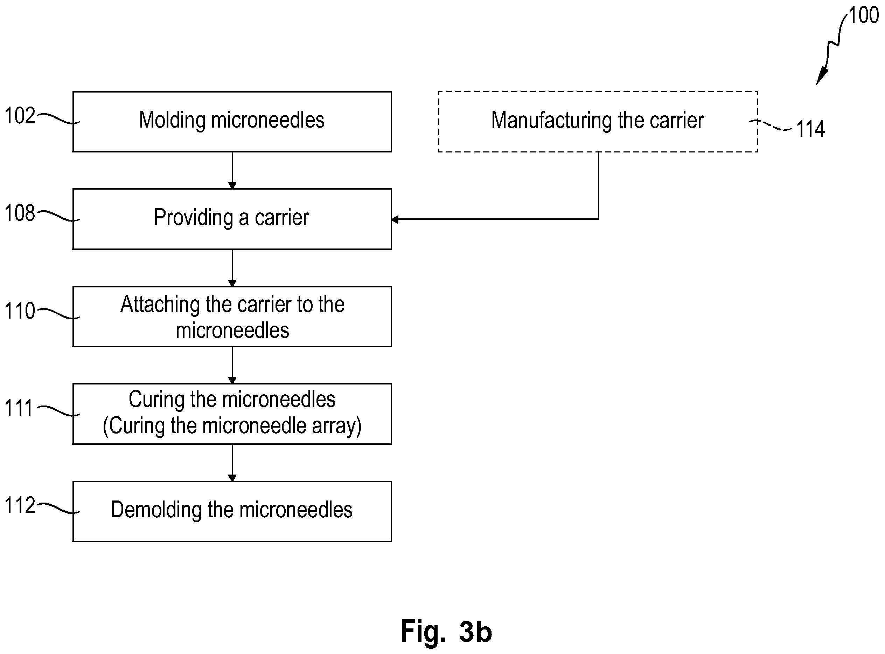

Figure 3b shows a flow chart indicating another embodiment of a method 100 for manufacturing a microneedle array 10.

The embodiment essentially corresponds to the embodiment of Fig. 3a.

In contrast to the embodiment of Fig. 3a, a further step of *curing the microneedies* 111 is performed after *attaching the carrier to the microneedles 110* and before *demolding the microneedles 112.* For example, the microneedles 12 can be cured by drying the microneedles 12 by applying heat, by applying gas flow, by applying UV light and/or by a curing time, for example 30 mins.

Fig. 3b particularly indicates a method whereby the microneedles 12 are attached to the carrier 14 by a diffusion bonded and/or welded joined connection, since curing of the microneedles 12 is performed after connecting the carrier 14 to the microneedles 12.

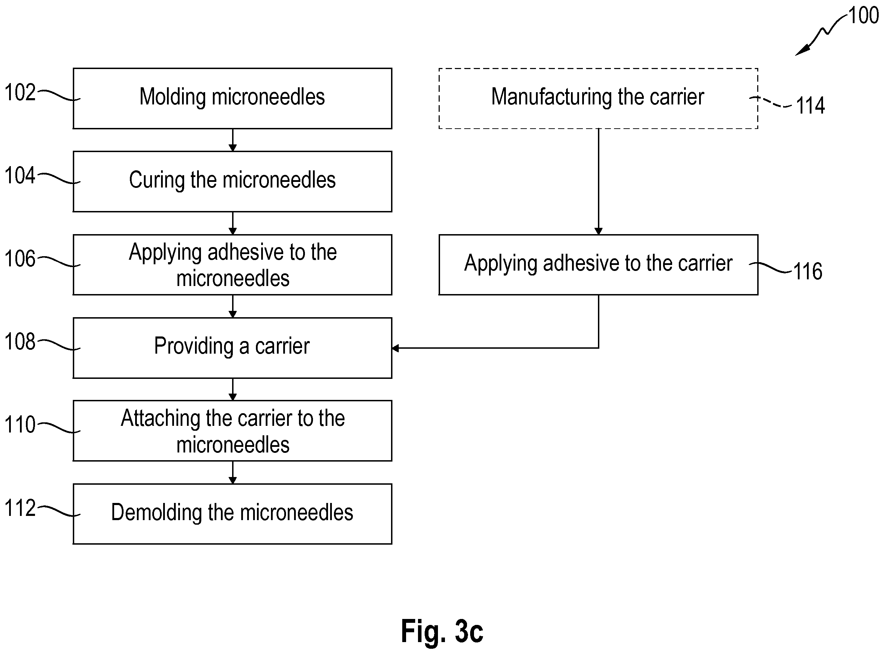

Figure 3c shows a flow chart indicating another embodiment of a method 100 for manufacturing a microneedle array 10.

The embodiment essentially corresponds to the embodiment of Fig. 3a.

In contrast to the embodiment of Fig. 3a, a further step of *curing the microneedies* 104 is performed after *molding the microneedles 110.* Additionally, the further steps of *applying adhesive to the microneedles 106* after *curing the microneedles* 104 and of *applying adhesive to the carrier 116* after manufacturing the carrier 114 is shown.

*Attaching the carrier to the microneedles* 110 is thus achieved by an adhesive bond between the carrier 14 and the microneedles 12.

Instead of (as shown) applying adhesive to the microneedles 12 and the carrier 14 it is preferably alternatively possible to apply the adhesive only to the microneedles 12 or the carrier 14.

Further exemplary, preferred methods are indicated in the following:

### I. First exemplary, preferred method

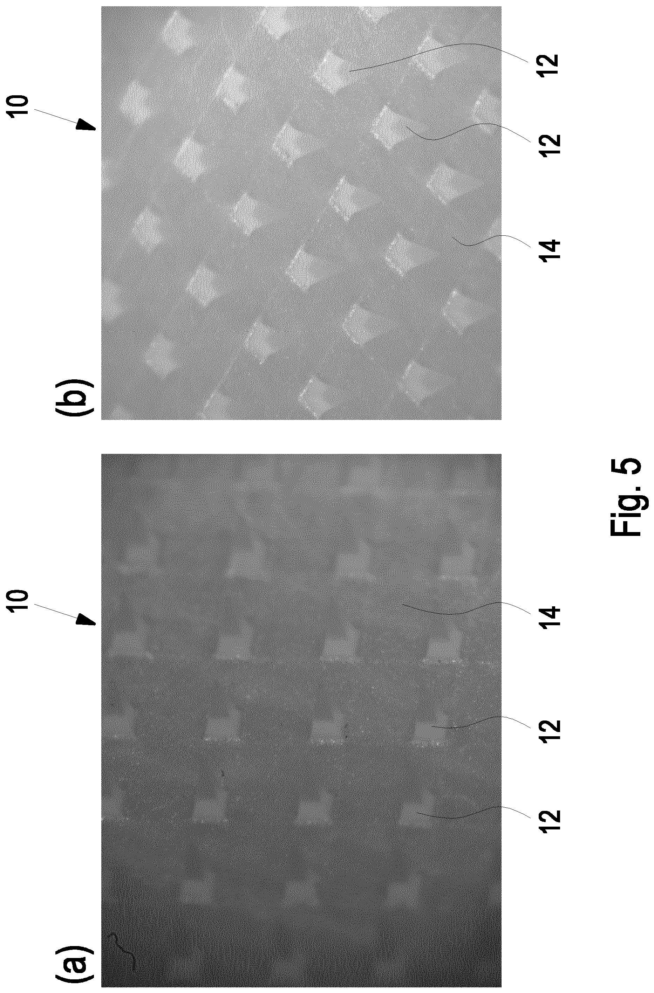

1.) Production of biocompatible carrier 14 with the dimensions of a backing: In this case, the carrier 14 are 3D printed using OBJET 260 CONNEX3 by Stratasys as a printer and MED610 as polymer. The carrier 14 is cylindrical, having a diameter of 10 mm and a height of 0.5 mm. The height of the carrier 14 should preferably be at least 0.2 mm or 0.3 mm.
2.) Positioning gas-permeable mold: A plurality of gas-permeable molds 16, particularly silicone matrices, are provided. A vacuum is applied to the mold 16 using a vacuum device, such as a pump. For example, as shown in Fig. 4a, the plurality of molds 16 can be provided in a mounting device 17, such as a mounting plate. The mounting device 17 particularly has a plurality of recesses 21 for receiving a mold 16 each. It is preferred that the recesses 21 have air openings (not shown), e.g. a grid structure, for applying the vacuum, so that the vacuum can be applied to the molds 16, preferably from the underside, through the recesses 21.
3.) Dosing the polymer-based formulation: A very thin layer of the first waterinsoluble compound for producing the microneedles 12 is dosed into the molds 16. This compound can be a hydrophobic polymer, for example. The geometry of the molds 16 and the use of vacuum draw the compound into the cavities 15, creating a thin outer layer. The vacuum helps to distribute the compound evenly and avoid air bubbles.
4.) It takes about 7 minutes to dose 49 molds on a single layer. Immediately after dosing, the pre-manufactured carriers 14 are placed on the dosed microneedles 12.
5.) After 30 minutes, the vacuum is switched off and the microneedles 12 are demolded by the carrier 14. Examples of the demolded microneedles are shown in Figs. 5a-b. Hereby, the carrier 14 together with the microneedles 12 connected to it, forms a microneedle array 10.

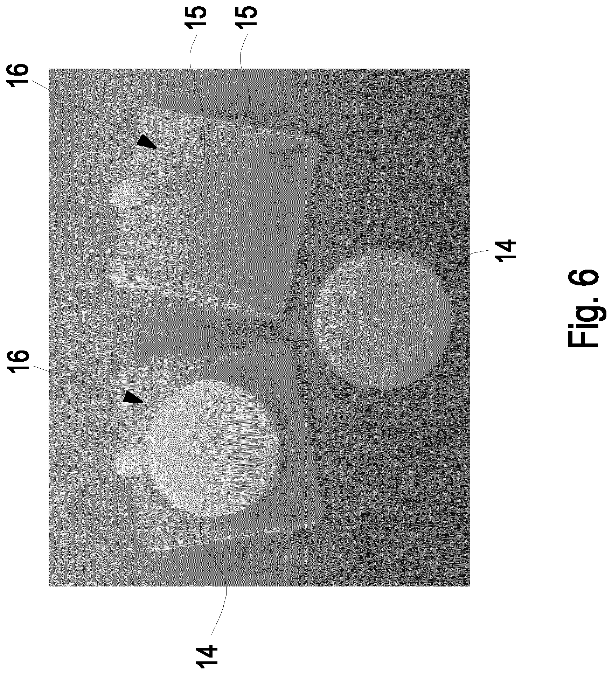

Fig. 6 shows an example of a mold 16 (top right), a carrier 14 (bottom) and a mold 16 with an attached carrier 14 (top left).

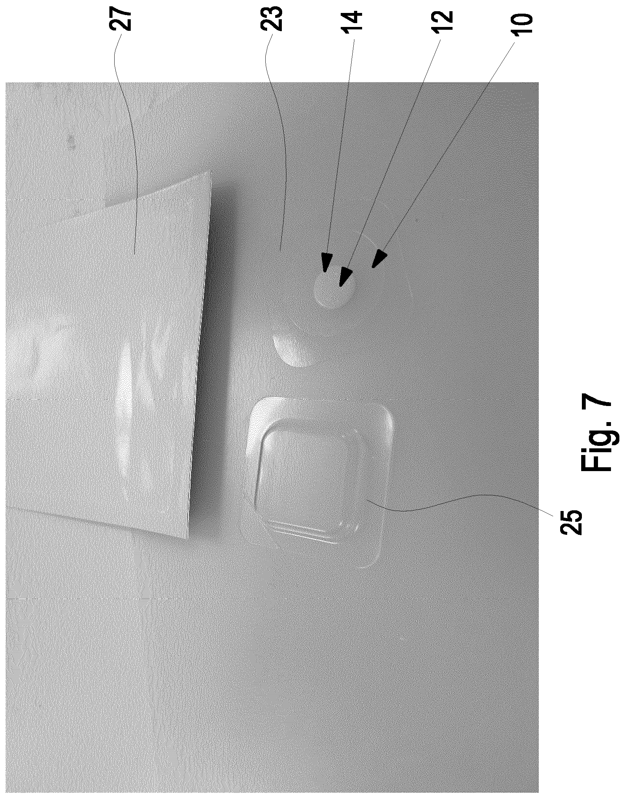

As exemplarily indicated in Fig. 7, after production, the carrier 14 with the connected microneedles 12 can be attached to a patch 23. Here, the patch with the connected carrier 14 forms the microneedle array 10. The microneedle array 10 can preferably be stored stably in a blister 25. The blister 25 holding the microneedle array 10 may be packaged in a sealed pouch 27.

### II. Second exemplary, preferred method

Process description: production and use of the carrier 14 for demolding microneedles 12.
1. Starting point: individual dosing in a mold 16:
   The mold 16 is filled with a biocompatible material (e.g. PLGA, PVP or dextran) in its cavities 15.
   This mold 15 is used to initially form the microneedles 12 and then to demold them using the carrier 14.
2. Methods for producing the carrier 14
   A. Production as a large mat (batch process)
      i. Large-scale production:
         The carrier 14 is initially produced as a large, homogeneous mat made of a suitable material.
         Materials such as PLA, PLGA or PCL can be used here. Procedure:
            The carrier 14 may, for example, be manufactured by injection molding.
            The mat can be provided with special microstructures/adhesive layer to optimize interaction with the microneedles.
      ii. Microneedle 12 demolding:
         The large mat is placed on the mold 16 with the microneedles 12 and activated by pressure or heat to safely demold the microneedles 12.
      iii. Punching into individual carriers 14:
         After the microneedles 12 have been demolded, the large mat is punched into smaller, standardized carriers 14 that can be inserted directly into microneedle patches or into an applicator.
         Advantages of this method:
            High efficiency through batch production.
            Low material loss during punching.
            Precise control of carrier 14 size and shape.
   B. Production of individual carriers 14 (direct process)
      i. Individual molding:
         Each carrier 14 is directly manufactured in the required form,
            e.g. as a cylindrical carrier 14 with the appropriate size.
         Process:
            the carrier 14 may, for example, be manufactured by injection molding
      ii. Microneedle demolding:
         The carrier 14 is placed directly on the filled microneedle matrix.
         Pressure, heat or other mechanisms are used to release the material of the microneedles.
         Advantages of this method:
            No additional processing steps (e.g. punching).
            High precision in the production of the carrier 14s.
      iii. Use of the carrier 14 to release microneedles 12
         Regardless of the method (mat or single carrier 14), the demolding process can be as follows:
         The carrier 14 is brought into contact with the filled mold 16.
         Mechanisms for demolding:
            Pressure: The carrier 14 presses the microneedles 12 out without damaging the mold 16.
            Heat: A controlled heat pulse can help to separate the microneedle material from the mold material.
            Adhesion: The carrier 14 can be coated so that the microneedles 12 adhere to it better than to the matrix.
            After the demolding step, the microneedle structure remains securely on the carrier 14.
            The carrier 14s with the microneedles 12 can be integrated directly into a patch system or into the applicator.

### Testing the mechanical stability and insertion ability of a microneedle array

In order to demonstrate that the pre-manufactured carrier enables good mechanical support for the plurality of microneedles connected thereto and improved insertion ability, two tests are standardly performed, i.e. pressure test and insertion test.

The pressure test is 3-staged and has a first phase where the sample is compressed with a low force of 20N to make it even, then the sample is moved 1,2mm against the microarray measuring the resistance force and then again pressed with 20N to detect the resulting deformation. 1,2mm displacement is larger than the actual needles, however the force is measured by a bending beam and a part of the deformation goes into the beam. The required thresholds are typically at least 250N and maximum 280µm deformation.

For the insertion test applied forces are typically in the range of 32N, 50-55N or even above 100N depending on the design. The silicone membrane has a shore A hardness of about 45° and the foil is 35µm thick. Penetration is successful, if the needle pattern is visible under a microscope. The microscope used is a Keyence 3D Laser scanning microscope VK-X1000. The parameters for the measurement are defined automatically by the software and the magnification used is usually 20x or 100x. Furthermore, it could be selected either the laser scanning or confocal microscopy procedure.

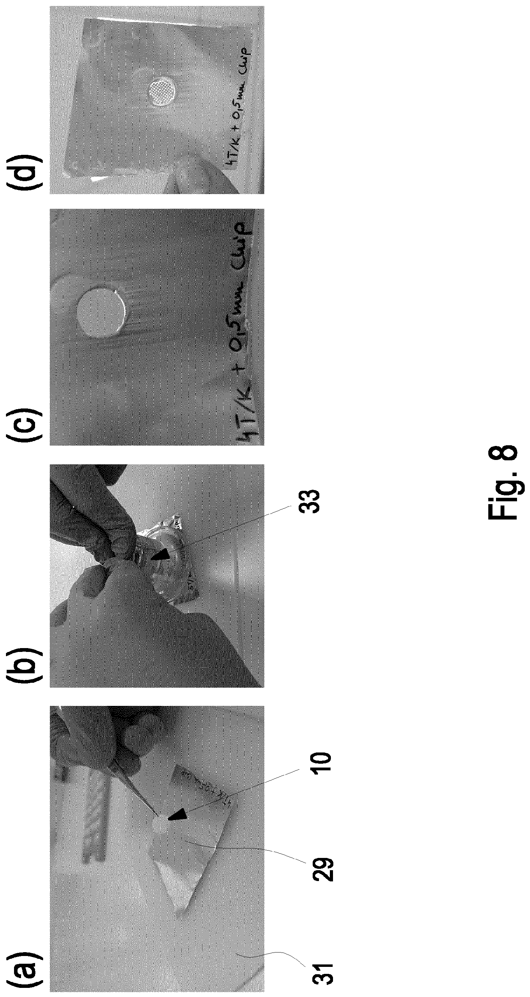

Results of the tests are shown in Figs 8-9.

Fig. 8a shows microneedle array 10 with microneedles 14 connected to a carrier 14, particularly according to Figs. 4-5. The microneedle array 10 is placed on aluminum foil 29 on a silicone mat 31, whereby the silicone mat 31 imitates a skin model.

Next, as shown in Fig. 8b, the microneedle array 10 is applied using an applicator 33.

The results after application are indicated in Figs. 8c and 8d. Fig. 8c shows the intact carrier after application. Fig. 8d shows the penetration of the microneedles 14 into the aluminum foil 29.

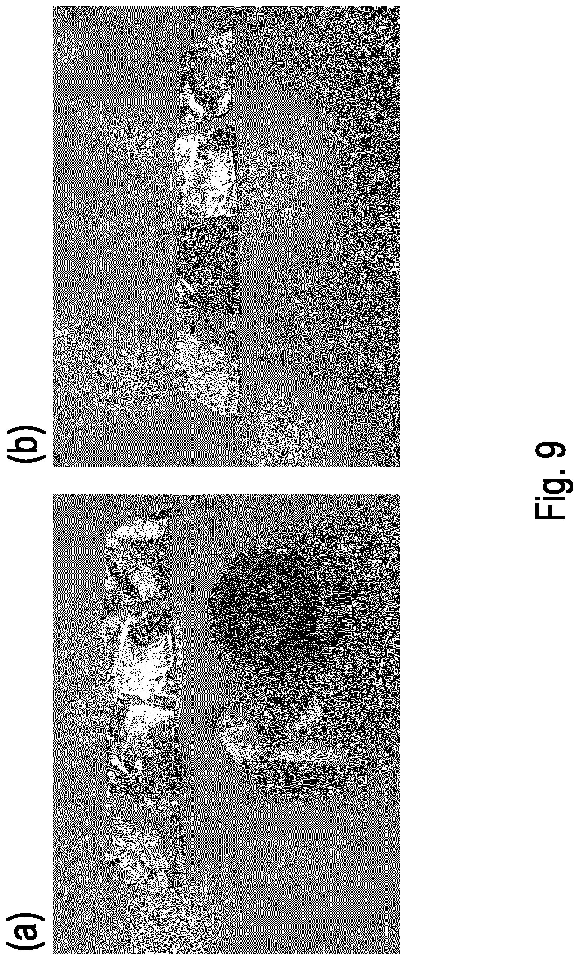

Further results are shown in Figs. 9a and 9b.

## Claims

1. Method for manufacturing a microneedle array (10) comprising a carrier (14) and a plurality of microneedles (12) connected to the carrier (14), the method comprising the steps of:
- molding the plurality of microneedles (12) in a, preferably gas-permeable, mold (16),
- providing the, preferably solid, carrier, and
- attaching the carrier (14) to the plurality of microneedles (12) in the mold (16).

2. Method according to claim 1, wherein the carrier (14) is attached to the, preferably cured, microneedles (12) by an adhesive layer (24) on the microneedles (12) and/or on the carrier (14).

3. Method according to claim 1 or 2, wherein the step of attaching the carrier (14) to the microneedles (12) comprises the step of bringing the carrier (14) into contact with the microneedles (12) in the mold (16), in particularly by pressing the carrier (14) onto the microneedles (12).

4. Method according to any one of claims 1-3, wherein the method comprises the further step of demolding the microneedles (12) from the mold (16) by removing the carrier (14) with the attached microneedles (12) from the mold (16).

5. Method according to any one of claims 1-4, wherein the carrier (14) is attached to the microneedles (12) by connecting the carrier (14) to the microneedles (12) while the microneedles (12) are in a liquid state.

6. Method according to any one of claims 1-5, wherein the carrier (14) is attached to the microneedles (12) by diffusion bonding and/or welded joining.

7. Method according to any one of claims 1-6, wherein the carrier (14) is a backing plate having a rectangular, square, round, or oval shape.

8. Method according to any one of claims 1-7, wherein the carrier (14) has a thickness of 50 µm to 1000 µm, preferably of 100 µm to 500 µm and more preferably of 300 µm to 500 µm.

9. Method according to any one of claims 1-8, wherein the method comprises the further step of manufacturing the carrier (14) by cutting the carrier (14) from a base layer, particularly a film; or by molding, particularly injection molding; or by 3D printing.

10. Method according to any one of claims 1-9, wherein a vacuum (18) is applied to the mold (16) at least during the step of molding the plurality of microneedles.

11. System for manufacturing a microneedle array (10), particularly using the method of any one of claims 1-10, the system comprising:
a, preferably gas-permeable, mold (16) for molding microneedles (12),
a device for attaching a carrier (14) to the microneedles (12) in the mold (16).

12. System according to claim 11, wherein the device for attaching the carrier (14) is adapted to demold the microneedles (12) by removing the carrier (14) from the mold (16) with the microneedles (12) attached to the carrier (14).

13. System according to claim 12, wherein the system further comprises a vacuum device for applying a vacuum (18) to the mold (16).

14. A carrier (14) for providing a mechanical support for a plurality of microneedles (12) connected thereto,
wherein the carrier (14) is pre-manufactured and obtainable by process of cutting the carrier (14) from a base layer, particularly a film; or by molding, particularly injection molding; or by 3D printing.

15. The carrier (14) according to claim 14, wherein the carrier (14) is a backing plate having a rectangular, square, round, or oval shape.

16. The carrier (14) according to claims 14 or 15, wherein the carrier (14) has a thickness of 50 µm to 1000 µm, preferably of 100 µm to 500 µm and more preferably of 300 µm to 500 µm.

17. The carrier (14) according to any one of claims 14-16, wherein the carrier comprises a biocompatible material.

18. The carrier (14) according any one of claims 14-17, wherein the biocompatible material is selected from dextran, polyvinylpyrrolidone (PVP), gelatin, polylactide-co-glycolide (PLGA), polylactic acid (PLA), polyhydroxyalkanoates (PHA), polyglycolic acid (PGA), polycaprolactone (PCL), cellulose acetate, chitosan, alginate, polyethylene terephthalate (PET), MED625FLX, MED610 and/or MED620.

19. Microneedle array (10), particularly manufactured by a method according to any one of claims 1-13, comprising:
a carrier (14), particularly a carrier according to any one of claims 14-18, and
a plurality of microneedles (12),
wherein the microneedles (12) are attached to the carrier (14), preferably by adhesive, diffusion, or welded joints.

20. Microneedle array (10) according to claim 14 or method according to any one of claims 1-14, wherein the microneedles (12) are dissolving microneedles (12) or hydrogel microneedles (12).
